# EUROPEAN PATENT APPLICATION

(11) **EP 3 343 489 A1**
(43) Date of publication of application: **04.07.2018**
(21) Application number: 16306839.8
(22) Date of filing: 29.12.2016
(51) Int. Cl.: G06Q 30/02, G06Q 50/22, G06Q 50/10, G06Q 10/00, G06Q 50/00, G06F 11/34

(54) **METHOD AND APPARATUS FOR DETECTING USER INTERACTIONS WITH A COMMUNICATION DEVICE**

(71) Applicant: Thomson Licensing, 92130 Issy-les-Moulineaux (FR)
(72) Inventor: LE BOLZER, Françoise, 35576 Cesson-Sévigné (FR); VIGOUROUX, Jean-Ronan, 35576 Cesson-Sévigné (FR); MAY, Martin, 35576 Cesson-Sévigné (FR); HEEN, Olivier, 35576 Cesson-Sévigné (FR)
(74) Representative: Huchet, Anne

(57) **Abstract**

The method and apparatus utilize communication devices and systems and/or Internet of Things (IoT) devices and systems to track a person's user interaction events, store them, and process the individual events or the accumulated history of these events for computing and generating an indicator of the level of user isolation for the tracked individual. During the monitoring and processing of the events, when the generated indicator meets or exceeds a predetermined value, an alert can be generated to notify the individual's friends, relatives, associates, or other identified persons to initiate contact with the senior.

## Description

### FIELD

The disclosure relates to tracking user interaction via communication devices and, more particularly, to a method and apparatus for detecting, for example a level of activity or inactivity and for establishing an indicator whose value can be used to initiate an alert. The disclosure further relates to a gateway comprising such an apparatus.

### BACKGROUND OF THE DISCLOSURE

Monitoring the activity or inactivity of a person or persons may have many useful applications. For example, as the senior and especially the elderly segments of the world's population continue to increase in age, they are faced with potential physical and cognitive degradation that can limit mobility and possibly restrict active social activity. According to some research, there is a connection between cognitive decline and reduced social interaction and social connectivity. That is, research study groups have found that an elderly or senior person that lacks or has limited social interactions and connections can experience a noticeable cognitive decline at a much faster rate than a similarly situated person that has many more social interactions.

For ease of reference in the presentation below, the terms "elderly" and "senior" are used interchangeably and are intended to convey the same meaning. Other monitoring or surveillance applications of person activity or inactivity can also be envisaged.

### SUMMARY

The disclosure takes advantage of communication systems and devices or/and other Internet of Things (IoT) devices and systems. According to a first aspect of the disclosure a method is described in which a first activity value is generated based on interactions of a user with one or more devices associated with such user's profile. An alert is than provided based on a comparison of the first activity value to a second activity value generated during a particular period of time.

In another embodiment, the second activity value is based on previous monitoring of said interactions of a user with one or more devices associated with said user's profile.

In another embodiment, the alert is provided when the second activity value is the same or greater than said first activity value in said comparing operation.

In another embodiment, providing the alert includes transmitting a notification to one or more individuals identified in said user's profile to initiate contact between said one or more individuals and said user.

According to another aspect of the disclosure, an apparatus is described having a memory that stores a plurality of instructions and a processor coupled to the memory. The memory is configured to execute the instructions to generate a first activity value based on interactions of a user with one or more devices associated with said user's profile, and to provide an alert based on a comparison of the first activity value to a second activity value generated during a period of time.

In another embodiment, the second activity value is based on previous monitoring of said interactions of a user with one or more devices associated with said user's profile.

In another embodiment, the alert is provided when the second activity value is the same or greater than said first activity value in said comparing operation.

In another embodiment, providing the alert includes transmitting a notification to one or more individuals identified in said user's profile to initiate contact between said one or more individuals and said user.

According to another aspect of the disclosure, a gateway is provided having a memory that stores a plurality of instructions and a processor coupled to the memory. The memory is configured to execute the instructions to generate a first activity value based on interactions of a user with one or more devices associated with said user's profile, and to provide an alert based on a comparison of the first activity value to a second activity value generated during a period of time.

At least parts of the methods of the disclosure may be computer implemented. Accordingly, embodiments may take the form of an entirely hardware embodiment, an entirely software embodiment (including firmware, resident software, micro-code, etc.) or an embodiment combining software and hardware aspects that may all generally be referred to herein as a "circuit", "module" or "system". Furthermore, the embodiments may take the form of a computer program product embodied in any tangible medium of expression having computer usable program code embodied in the medium.

Since embodiments can be implemented in software, at least parts of the methods can be embodied as computer readable code for provision to a programmable apparatus on any suitable carrier medium. A tangible carrier medium may comprise a storage medium as a floppy disk, a CD-ROM, a hard disk drive, a magnetic tape device or a solid-state memory device and the like. A transient carrier medium may include a signal such as an electrical signal, an electronic signal, an optical signal, an acoustic signal, a magnetic signal or an electromagnetic signal, e.g., a microwave or RE signal.

### BRIEF DESCRIPTION OF THE DRAWINGS

The teachings of the present invention can be readily understood by considering the following detailed description in conjunction with the accompanying drawings, in which:
FIG. 1 illustrates a system block diagram of an exemplary environment in which embodiments of the present method and apparatus may be implemented;
FIG. 2 illustrates a simplified block diagram of an embodiment of an apparatus configured to perform the method illustrated in FIG. 3; and
FIG. 3 illustrates the operations performed in accordance with an embodiment of the present method.

It should be understood that the drawings are for purposes of illustrating the concepts of the invention and are not necessarily the only possible configuration for illustrating the invention. To facilitate understanding, identical reference numerals have been used, where possible, to designate identical elements that are common to the figures.

### DETAILED DESCRIPTION

Embodiments of the present method and apparatus may be directed to tracking user interaction via communication devices in a substantially independent living environment and, thereby, detecting, for example, a level of social interaction and potential loneliness in people in order to generate an indicator whose value can be used to initiate an alert under certain conditions. Although the description below may focus predominately on independent living applications, it will be understood that the present methods and apparatus are equally applicable in dependent living facility such as a nursing home, hospital or the like where social interaction is expected and possible. Moreover, it will be understood that the present method and apparatus may be applied to other tracking applications outside elderly care.

"Isolation" or even "social connectedness" can be measured objectively and they can be used as indicators of loneliness. As a person's social interactions decrease in number and frequency, they can be understood to connote a higher degree of isolation and, concomitantly, a lower degree of social connectedness. A person who is more isolated and thereby less socially connected can experience a certain amount of loneliness. In this description, these terms may all be used synonymously and interchangeably without any loss of generality and meaning.

With the growth of various Internet technologies, individuals are able to maintain social connections via IP-based communication systems. These technologies involve telephony, social networks, and videoconference, to name just a few. They are widely available via applications running on computers and cell phones. In our senior/elderly population, these communications tools and systems are rendered useless when the elderly do not contact their relatives for fear of disturbing them or because they forget or do not even think to contact them.

Embodiments of the present arrangement provide a method and apparatus for monitoring social isolation for the tracked individual such as, for example, a senior or elderly person and formulating an indicator that is then used to provide an alert when the indicator identifies the detection of more than a predetermined level of social isolation. In one embodiment of the method and apparatus, the communications systems or/and other IoT facilities are utilized to track the senior's user interaction events, store them in a database and process their history to manage an indicator. When the indicator reaches or exceeds a particular threshold value or when the indicator remains at or above the threshold level during a given period of time, an alert may be provided to engage the senior's relatives to initiate a contact. In response to the alert, a communication system can then initiate communication between the senior person and a peer according to knowledge of the peer's availabilities.

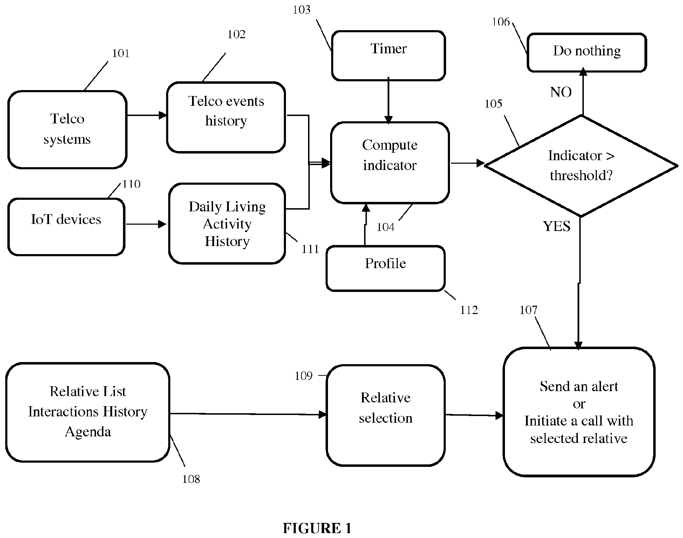

FIG. 1 illustrates a system block diagram of an exemplary environment in which the present method and apparatus are intended to operate. As depicted in FIG. 1, the generation of the indicator and generation of an alert for isolation can involve the operations of exemplary elements 104-107 with inputs from exemplary elements 101-103 and 108-111. The operational and input elements/systems, including the databases or lists shown in FIG. 1, are intended to be exemplary and are not intended to be limiting to realization of the structure of the present apparatus and operations of the present method. Moreover, it is contemplated that certain elements, such as the timer and other input (list) elements, can be incorporated into the structure of the present apparatus.

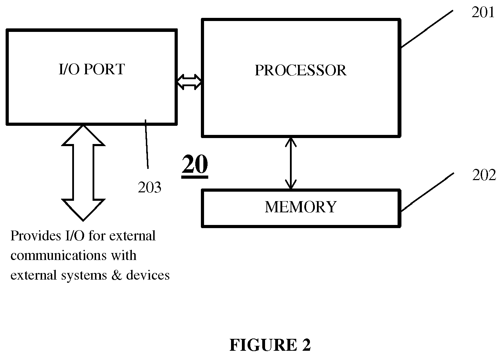

An exemplary apparatus 20 is depicted in Figure 2. This apparatus is configured to perform at least the computation of the indicator and generation of an alert for isolation involving the operations of exemplary elements 104-107 in response to inputs from exemplary elements 101-103 and 108-111. Apparatus 20 includes a processor 201, a memory 202, and an input/output port 13. Processor 201 is coupled to memory 202, which stores information and instructions that are capable of being used and executed by the processor. The processor 201 communicates with external devices and systems through the I/O port 203. I/O port 203 provides a communicative coupling between the apparatus 20 and external devices/systems such as those shown in FIG. 1 together with any additional devices providing input concerning social interaction events for the tracked individual. The I/O port is used for sending alerts and notifications, for example. I/O port 203 is also used for receiving social interaction events for the tracked individual.

Apparatus 20 receives various input information in order to compute the indicator of social isolation and to perform the threshold comparison required for generating the alert. All the social interaction events for an individual can be aggregated in a central device such as a home gateway or another exemplary device such as device 20 in FIG. 2. The events can be stored and processed in order to compute the indicator and generate the alert.

As shown in FIGs. 1 and 2, inputs are received from various data sources. Telecommunications system 101, IoT (i.e., Internet of Things) devices 110, timer 103, profile 112, and additional information included in exemplary element 108 provides multiple sources of input for computation of the indicator and generation of the alert. Element 108 can provide additional devices, systems, and information pertinent to tracking an individual of interest.

Broadly interpreted and contemplated herein, telecommunication systems 101 are intended to include elements that provide direct information about the social activity and connections of the senior person. Such systems include, but are not limited to landline and cellular telephone systems; short messaging service (SMS) based systems including text messaging, Twitter, and the like; e-mail systems; video conferencing service systems including dedicated systems and even applications such as Skype and Google's Hangouts and the like; and social networking platforms such as Facebook and the like. To varying degrees, each of these systems can provide various amounts and types of information about the social activities and interactions of a particular individual.

IoT devices 110 can also provide information from which a certain level of social activity can be extracted or inferred. IoT devices are realized in some cases as sensors deployed in the home. This form of IoT device 110 includes but is not limited to microphones, cameras, motion detectors, and door contactors, for example. Other IoT devices 110 are wearable devices that can be worn by the person being tracked. This latter type of IoT device 110 can be realized as a connected wristband device, a mobile phone, Google glasses, and the like.

Yet another type of IoT device includes a smart device running an embedded application, wherein the smart device is one of a tablet, a laptop computer, a mobile phone and the like. These smart devices and their embedded applications can collect and provide information about daily life activity and habits of the tracked individual senior.

The IoT devices can monitor and measure social interaction, whether via in-person face-to-face or on user networks. They can also detect visitors for the tracked individual. As an example of the daily life activity monitoring of a resident, motion detectors and door contactors can be advantageously used to detect periods during which the tracked individual is outside a particular location such as a room or a building, all reflective of a certain degree of social interaction.

As a user interaction event occurs or fails to occur, the indicator value can be increased or decreased. In exemplary computations, the indicator value can be decreased as soon as each new user interaction event is detected and it can be increased when a social interaction event fails to occur within a certain period of time. Timer 103 can be used to provide time of day and duration of time, for example, in the computation of the indicator.

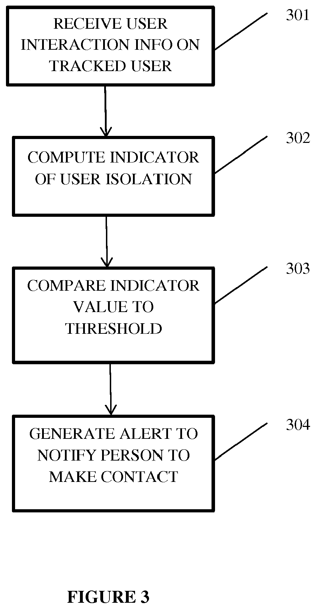

Certain data can be provided by the various systems or devices shown in FIG. 1 (see step 301 in FIG. 3). For example, telecommunications companies have historical information 102 regarding the calls made to and from the tracked individual, the party calling or called, the duration of the call, and date of the call. Similar information in historical information 102 may also pertain to text messages sent and received by the telecommunication provider. With respect to the IoT devices, the historical information 111 can contain information related to daily living activity such as frequency and length of walking and exercise routines or deep and light sleep patterns, for example.

Additional information can be maintained in a profile 112 or in other listings such as listing/database 108. The profile may include information about the tracked persons and individuals associated with that person. It may also include static threshold values for the indicator computation and comparison. The profile may also include a list of contacts, a preferential ranking of those contacts, and other information about the contacts including locations, availability, and contact phone numbers. Listing 108 can include information about relatives of the tracked person similar to the contact information in profile 112. Listing 108 may also be included within profile 112. This listing may also include an historical perspective on social interactions between each listed relative and the tracked individual.

For the indicator computation performed in element 104, the amount of increase/decrease for the indicator value can be defined statically by the tracked individual or relatives or others associated with the individual. Alternatively, the amount of increase/decrease for the indicator value can be set automatically based on an analysis of the nature of each user interaction event and its characteristics such as its duration, frequency, time of occurrence, and the like. In the latter case, each event may be classified in order to weight its contribution in the computation of the indicator. That is, some events or event characteristics can have a greater or lesser effect on the amount of indicator value change based on the occurrence/non-occurrence of a user interaction event. For example, it is contemplated that lack of user interaction events during an expected period of sleeping such as between 10 PM and 8 AM will have less of an effect in decreasing the indicator value than a similar lack of user interaction events during expected hours of wakefulness. In a similar vein, interaction via email or social networking sites such as Facebook may be envisioned as classifications having a lesser degree of impact on the indicator value than actual interpersonal interaction events. Other exemplary classifications may be considered such: the event originator, which would be used to balance interactions initiated by relatives against those initiated by the tracked individual; the outcome of the interaction, which could be used to identify whether a potential interaction such as a received email ended successfully with email opening and answer, for example; and the interaction event duration, which allows for differentiation between a brief "hello" and a long conversation, for example. Furthermore, the contribution of the event in the indicator computation may be weighted according to its frequency and its periodicity in the event history for the tracked individual.

When an event occurs (or fails to occur at a certain time or in a time period), the indicator value is updated in the apparatus in element 104 (see step 302 in FIG. 3). At that point, the indicator value is compared to a threshold value in the apparatus in element 105 (see step 303 in FIG. 3). When the indicator does not meet or exceed the threshold value, the apparatus in element 106 does not generate an alert and returns to monitoring social interaction events for computing and updating the indicator value. When the threshold value is met or exceeded, a sufficiently large amount of social isolation is thought to have occurred. In response to the condition being met in the threshold comparison, an alert is generated in element 107 to cause a notification to be transmitted to one or more individuals identified in the tracked person's profile or other listing to initiate contact between those individual(s) and the tracked person (see step 304 in FIG. 3). The notification can be transmitted directly from the apparatus 20 or it may be sent in response to the alert via one of the service providers for systems 101 and devices 110 and the like. For example, the alert may notify the relative or associate to contact the tracked person or it may initiate a call between a listed/selected party and the tracked person.

As shown in FIG. 1, relative selection element 109 in the apparatus can receive the list of relatives or other such contacts and select one or more individuals from the list for making contact with the alert notification. The selection can be based on factors such as a preference for making contact with the tracked person or for receiving alerts or an individual's availability at the particular time of an alert or any other such static or dynamic set of criteria. The selection is passed along to the element 107 in the apparatus for preparing the alert.

The functions of the various elements shown in the figures can be provided through the use of dedicated hardware as well as hardware capable of executing software in association with appropriate software. When provided by a processor, the functions can be provided by a single dedicated processor, by a single shared processor, or by a plurality of individual processors, some of which can be shared. Moreover, explicit use of the term "processor" or "controller" should not be construed to refer exclusively to hardware capable of executing software, and can implicitly include, without limitation, digital signal processor ("DSP") hardware, read-only memory ("ROM") for storing software, random access memory ("RAM"), and non-volatile storage. Moreover, all statements herein reciting principles, aspects, and embodiments of the invention, as well as specific examples thereof, are intended to encompass both structural and functional equivalents thereof. Additionally, it is intended that such equivalents include both currently known equivalents as well as equivalents developed in the future (i.e., any elements developed that perform the same function, regardless of structure).

Thus, for example, it will be appreciated by those skilled in the art that the block diagrams presented herein represent conceptual views of illustrative system components and/or circuitry embodying the principles of the invention. Similarly, it will be appreciated that any flow charts, flow diagrams, state transition diagrams, pseudo-code, and the like represent various processes which may be substantially represented in computer readable media and so executed by a computer or processor, whether or not such computer or processor is explicitly shown.

Having described various embodiments for a method for detecting social isolation, it is noted that modifications and variations of the method can be made by persons skilled in the art in light of the above teachings. It is therefore to be understood that changes may be made in the particular embodiments of the invention disclosed which are within the scope and spirit of the invention. While the forgoing is directed to various embodiments of the present invention, other and further embodiments of the invention may be devised without departing from the basic scope thereof.

## Claims

1. A method, comprising:
generating a first activity value (302) based on interactions of a user with one or more devices associated with said user's profile (301); and
providing an alert (304) based on a comparison of the first activity value to a second activity value (303) generated during a period of time.

2. The method defined in claim 1 wherein the second activity value is based on monitoring said interactions of a user with one or more devices associated with said user's profile.

3. The method as defined in claim 1 or 2, wherein said alert is provided when the second activity value is met or exceeded by said first activity value in said comparing operation.

4. The method as defined in any of claims 1 to 3, wherein providing said alert includes transmitting a notification to one or more individuals identified in said user's profile to initiate contact between said one or more individuals and said user.

5. Apparatus (20) comprising:
a memory (202) storing a plurality of instructions; and
a processor (201) coupled to the memory (202) and configured to execute the instructions to:
generate a first activity value based on interactions of a user with one or more devices associated with said user's profile; and
provide an alert based on a comparison of the first activity value to a second activity value generated during a period of time.

6. The apparatus of claim 5, wherein the second activity value is based on monitoring said interactions of a user with one or more devices associated with said user's profile.

7. The apparatus of claims 5 or 6, wherein said alert is provided when the second activity value is met or exceeded by said first activity value in said comparing operation.

8. The apparatus of any of claims 5 to 7, wherein providing said alert includes transmitting a notification to one or more individuals identified in said user's profile to initiate contact between said one or more individuals and said user.

9. A non transitory computer readable medium having one or more executable instructions stored thereon, which when executed by a processor cause the processor to perform a method according to any one of claims 1 to 4.

10. A computer program product for a programmable apparatus, the computer program product comprising a sequence of instructions for implementing a method according to any one of claims 1 to 4 when loaded into and executed by the programmable apparatus.

11. A gateway device comprising the apparatus of any one of claims 5 to 8 or the non transitory computer readable medium of claim 9.
